# EUROPEAN PATENT APPLICATION

(11) **EP 2 845 490 A1**
(43) Date of publication of application: **11.03.2015**
(21) Application number: 13275202.3
(22) Date of filing: 05.09.2013
(51) Int. Cl.: A23D 9/02, A23D 9/00, A23L 1/29, A61K 31/215, A61P 3/04

(54) **Fat composition for improved body fat distribution**

(71) Applicant: LODERS CROKLAAN B.V., 1521 AZ Wormerveer (NL)
(72) Inventor: Luisa GAMBELLI, 1521 AZ Wormerveer (NL); Banni, Sebastiano, 09010 Pula (CA) (IT)
(74) Representative: Stevens, Ian Edward

(57) **Abstract**

A composition for use in increasing the ratio of fat in subcutaneous adipose tissue to fat in visceral adipose tissue comprises a glyceride having a palmitoyl group at the 2-position wherein at least 40% by weight of the palmitic acid content of the composition is present as palmitoyl groups bonded at the 2- position of a glyceride.

## Description

This invention relates to a composition for use in increasing the ratio of fat in subcutaneous adipose tissue to fat in visceral adipose tissue, particularly in infants.

Dietary fat plays a fundamental role in human nutrition at different ages. More often dietary fat has been linked to its role as an energy substrate and deposit on account of its capacity to be stored in adipose tissue. However, it is now well recognised that not all adipose tissue is the same. Humans, and mammals in general, accumulate fat mainly in subcutaneous adipose tissue (SAT), typically about 80%, while a smaller fraction, 20%, accumulates in visceral adipose tissue (VAT). In certain physiological conditions when it is necessary to accumulate fat, as for example in infancy and in women because of pregnancy and lactation, it becomes particularly crucial to avoid excessive accumulation in visceral adipose tissue which may lead to energy and lipid metabolism impairment with consequent dyslipidaemia, insulin resistance, glucose intolerance and ectopic fat accumulation, all features of the so-called metabolic syndrome. In infancy this is particularly crucial because, while in women the relative high level of oestrogen favours accumulation in SAT, in infants it remains more problematic to avoid excessive accumulation in VAT.

Rolfe *et al,* Journal of Obesity, 2013, Article ID 951954 describes ultrasound estimates of visceral and subcutaneous-abdominal adipose tissues in infancy and discloses that post-natal nutrition may influence the accumulation of visceral adipose tissue in infancy.

Aguirre *et al,* Diabetology & Metabolic Syndrome, 2012, 4:35, relates to the effect of excess endocannabinoid anandamide (AEA) during lactation in inducing overweight, fat accumulation and insulin resistance in adult mice.

Blüher et al, Diabetes, 2006, 55(11):3053-3060, suggests that cannabinoid receptors in visceral adipose tissue may represent a primary target for the treatment of abdominal obesity and associated metabolic changes.

Patti and Corvera, Endocrine Reviews, 2010, 31 (3):364-395, discloses that mitochondrial biogenesis and function are decreased in white adipose tissue, liver, and skeletal muscle of obese/diabetic animals and humans.

Tedesco et al, Diabetes, 2010, 59:2826-2836, discloses that cannabinoid receptor stimulation impairs mitochondrial biogenesis in mouse white adipose tissue, muscle and liver.

Hoareau and Roche, Drug Discovery Today, 2010, 7(3-4):e205-e212, discloses that palmitoylethanolamide (PEA), in view of its anti-inflammatory properties on adipocytes, might have a role in obesity since, before its occurrence, the organism displays a low-grade inflammation of which adipocytes are probably largely responsible.

O'Sullivan and Kendall, Immunobiology, 2010, 215:611-818, reports that AEA activation of PPAR-gamma stimulates the differentiation of fibroblasts to adipocytes and discloses the role of OEA in lipolysis and inflammation.

Triglyceride fats and oils are important commercial products and are used extensively in, for example, the food industry. Some triglycerides are nutritionally important and the triglyceride 1,3-dioleoyl-2-palmitoyl glyceride (OPO) is known to be an important component of human milk fat.

Fat compositions containing the principal fatty acids found in human milk fat, in similar amounts to those found in human milk fat, may be derived from oils and fats of vegetable origin. However, a significant difference in composition arises because most glycerides of vegetable origin are unsaturated in the 2-position. In contrast, a substantial amount of palmitic acid occupies the 2-position of glycerides in human milk fat.

Innis, Advances in Nutrition, 2011, 2:275-283, describes the role of dietary triacylglycerol structure in infant nutrition.

Koletzko et al, Journal of Pediatric Gastroenterology and Nutrition, 2005, 41:584-589, describes a recommended composition for infant formula.

Processes are known for selectively producing the triglyceride 1,3-dioleoyl-2-palmitoyl glyceride (OPO) for use in replacements for human milk fat.

EP-A-0209327 discloses milk replacement fat compositions comprising the triglyceride 1,3-dioleoyl-2-palmitoyl glyceride (OPO). The fat compositions can be obtained by subjecting fatty mixtures of 2-palmitoyl glycerides to reaction with oleic acid in the presence of a catalyst, such as a lipase, which is regiospecific in activity in the 1- and 3-positions of the glycerides. Enzymatic processes of this kind are also described in GB-A-1577933. Under the influence of the catalyst, unsaturated fatty acid residues may be introduced into the 1- and 3- positions of the 2-palmitoyl glycerides by exchange with unsaturated free fatty acids or their alkyl esters.

WO 2005/036987 discloses a process for producing a fat base by reacting a palmitic rich oil with unsaturated fatty acids such as oleic acid. The total palmitic acid residue content of the fat base is at most 38% and at least 60% of the fatty acid moieties are in the 2-position of the glyceride backbone. A related disclosure can be found in WO 2005/037373, filed on the same day.

WO 2007/029018 describes a process for the production of a composition comprising 1,3-dioleoyl-2-palmitoyl glyceride (OPO) in which a palm oil stearin, with an iodine value (IV) between about 2 and about 12 is subjected to enzymatic transesterification, with oleic acid or a non-glyceride ester of oleic acid.

Yang et al, Maternal and Child Nutrition, 2013, 9, 105-119, (1) reports that most recent human studies support that breast-feeding is a protective factor for obesity/overweight.

Alvheim et al, Obesity, 2012, 20:1984-1994, discloses that dietary linoleic acid elevates endogenous 2-AG and anandamide and induces obesity.

Innis, Maternal and Child Nutrition, 2011, 7(2):112-123, suggests that omega-6 and omega-3 fatty acids, are bioactive nutrients, which in the short and potentially longer term regulate metabolic signalling and cellular differentiation, with the potential to increase or decrease lipid synthesis and storage.

There remains a need for compositions that can increase the ratio of fat in subcutaneous adipose tissue to fat in visceral adipose tissue. In particular, there is a need for compositions that can increase the ratio of fat in subcutaneous adipose tissue to fat in visceral adipose tissue whilst already being recognised as being safe for food use.

According to the invention, there is provided a composition comprising a glyceride having a palmitoyl group at the 2- position wherein at least 40% by weight of the palmitic acid content of the composition is present as palmitoyl groups bonded at the 2- position of a glyceride, for use in increasing the ratio of fat in subcutaneous adipose tissue to fat in visceral adipose tissue.

Also provided by the invention is the use of a composition comprising a glyceride having a palmitoyl group at the 2- position wherein at least 40% by weight of the palmitic acid content of the composition is present as palmitoyl groups bonded at the 2- position of a glyceride, for increasing the ratio of fat in subcutaneous adipose tissue to fat in visceral adipose tissue.

The invention also provides the use of a glyceride having a palmitoyl group at the 2-position in the manufacture of a composition for increasing the ratio of fat in subcutaneous adipose tissue to fat in visceral adipose tissue. Preferably, at least 40% by weight of the palmitic acid content of the composition is present as palmitoyl groups bonded at the 2- position of a glyceride.

Further provided by the invention is a method of increasing the ratio of fat in subcutaneous adipose tissue to fat in visceral adipose tissue in a subject, preferably an infant, comprising providing to said subject for consumption a composition comprising a glyceride having a palmitoyl group at the 2- position wherein at least 40% by weight of the palmitic acid content of the composition is present as palmitoyl groups bonded at the 2- position of a glyceride.

The invention relates to increasing the ratio of fat in subcutaneous adipose tissue to fat in visceral adipose tissue. Thus, the invention is concerned with the distribution of fat in the body. The invention is applicable to mammals. Preferably, the invention involves increasing the ratio of fat in subcutaneous adipose tissue to fat in visceral adipose tissue in humans, more preferably in infant humans (typically from 0 to 4 years old). However, the invention may involve increasing the ratio of fat in subcutaneous adipose tissue to fat in visceral adipose tissue in infants and in older humans.

In the invention, increasing the ratio of fat in subcutaneous adipose tissue to fat in visceral adipose tissue may involve, for example, a decrease in the fat in visceral adipose tissue and/or an increase in fat in subcutaneous adipose tissue. This can be achieved by the fat levels changing in a number of different ways. For instance, there may be a decrease in fat in visceral adipose tissue and a decrease in fat in subcutaneous adipose tissue with the decrease in the former being greater than the decrease in the latter. Alternatively, the level of fat in visceral adipose tissue may stay the same or increase but by not as much as the increase in the level of fat in subcutaneous adipose tissue.

The invention involves a surprising decrease in the level of anandamide (AEA) and an increase in the level of palmitoylethanolamide (PEA) in visceral adipose tissue. Preferably, the invention involves a decrease in the level of AEA in visceral adipose tissue of from 30 % to 70 %, more preferably from 40 % to 60 %, relative to a control not involving the composition of the invention. Additionally or alternatively, the invention preferably involves an increase in the level of PEA in visceral adipose tissue of from 10 % to 50 %, more preferably from 20 % to 40 %, relative to the control.

The total level of AEA, PEA and OEA in the visceral adipose tissue preferably remains roughly constant in the invention i.e., within plus or minus 10 % of its original level.

Surprisingly, palmitic acid in the sn-2 position in dietary glycerides has been shown to influence different biological functions. Data on rats on a diet rich in 2-pamitoyl glycerides showed a decrease of the endocannabinoid anandamide (AEA) and a concomitant increase of its congener palmitoylethanolamide (PEA) in visceral adipose tissue (VAT), and an increase of oleoylethanolamide (OEA) in muscle and liver. The decrease of AEA in the visceral adipose tissue (VAT) is expected to reduce triglyceride accumulation because 1) AEA is a cannabinoid receptor ligand which, once stimulated, impairs mitochondria function; 2) AEA activates PPAR-gamma transcriptional activity, known to stimulate pre-adipocyte differentiation and triglyceride accumulation thus circulating endocannabinoids have been associated to visceral abdominal obesity. Furthermore, the increase of PEA in VAT and OEA in muscle and liver may exert a systemic anti-inflammatory activity as demonstrated by the decrease of TNF-alpha in plasma after lipopolysaccharide (LPS) treatment, thereby providing an improved insulin sensitivity which may further favour dietary triglyceride accumulation in SAT rather than VAT.

It is believed that a change in the position of palmitoyl groups in dietary triglycerides triggers a series of modifications in endocannabinoids and congeners biosynthesis which may contribute in the physiological compartmentalization of adipose tissue, particularly in infants.

The term glyceride, as used herein, includes mono-, di- and tri- glycerides of fatty acids, and mixtures thereof, unless otherwise specified.

The term fatty acid as used herein refers to saturated or unsaturated, straight chain carboxylic acids having from 6 to 24 carbon atoms, preferably from 12 to 22 carbon atoms. Unsaturated acids may comprise one, two, or more double bonds, preferably one or two double bonds.

The glyceride used in the invention is preferably derived from vegetable sources, more preferably from palm oil. The glyceride is typically synthesised from palm oil or a fraction of palm oil. It will therefore be appreciated that the term derived from vegetable sources means that the glyceride is obtained directly or indirectly from vegetable sources and that the glyceride is not obtained directly or indirectly from animal products.

Preferably, the glyceride having a palmitoyl group is a triglyceride, more preferably 1,3-dioleoyl-2-palmitoyl glyceride (OPO). The OPO content of the composition is preferably from 1 % to 90 % by weight, more preferably from 5 % to 75 % by weight, even more preferably from 10 % to 50 % by weight, such as from 10 % to 30 % by weight. The composition may comprise other glycerides, such as other triglycerides.

The composition comprising the glyceride having a palmitoyl group at the 2- position has at least 40% by weight of the palmitic acid content of the composition present as palmitoyl groups bonded at the 2- position of a glyceride. A preferred range for the palmitic acid content of the composition present as palmitoyl groups bonded at the 2-position of a glyceride is from 40% to 60% by weight. The percentage of palmitic acid residues in the 2- position is preferably determined by finding: (a) the total palmitic content of the glycerides in the composition by FAME (fatty acid methyl ester analysis) (surface internal standard C17:0); and (b) the palmitic content of the 2- position by FAME (same standard) of a sample of the composition after hydrolysis of the 1- and 3- residues using pancreatic lipase to form a 2-monoglyceride. The value is ((b) multiplied by 100) ÷ ((a) multiplied by 3).

In one aspect of the invention, at least 45%, at least 50%, at least 60% or at least 70% by weight of the palmitic acid content of the composition is present as palmitoyl groups bonded at the 2- position of a glyceride.

It is believed that the present composition may increase availability of ALA for docosahexaenoic acid (DHA) synthesis in the developing infant, thus balancing the effects of linoleic acid (LA).

Optionally, the composition of the invention comprises alpha-linolenic acid (ALA) or a derivative thereof in an amount of greater than 1% by weight alpha-linolenic acid (in free and bound forms) based on total free and bound C₆ to C₂₄ fatty acids. More preferably, the amount of alpha-linolenic acid based on total free and bound C₆ to C₂₄ fatty acids in the composition of the invention is greater than 1.3%, such as from 1.3% to 2.0%, even more preferably greater than 1.5% by weight.

Derivatives of alpha-linolenic acid (ALA) or other fatty acids include non-toxic salts and esters, such as sodium salts, C₁ to C₆ alkyl esters (preferably straight chain) and mono-, di- and tri- glycerides. Glycerides are preferred derivatives.

The composition of the invention may comprise linoleic acid or a glyceride thereof. The weight ratio of total free and bound linoleic acid to total free and bound alpha-linolenic acid is preferably 15:1 or less, more preferably from 15:1 to 5:1. The amount of linoleic acid or glyceride thereof in the composition of the invention is preferably from 12 to 14% by weight based on total free and bound C₆ to C₂₄ fatty acids in the composition.

The terms free and bound refer to fatty acids that are either free carboxylic acids or their salts, or are covalently bonded to another group, for example as esters such as glycerides. For example, the total free and bound fatty acid (such as alpha-linolenic acid) in a glyceride composition includes the fatty acid present in mono-, di- and tri- glycerides. The total free and bound fatty acid content of a composition may be determined by FAME analysis.

The composition may be a blend comprising (i) a composition comprising alpha-linolenic acid or a glyceride thereof and (ii) a triglyceride composition comprising OPO in an amount of at least 20% by weight. The composition comprising alpha-linolenic acid or a glyceride thereof is preferably a vegetable oil. Preferred sources of alpha-linolenic acid are canola (rapeseed) oil, flaxseed oil and mixtures thereof.

The composition of the invention is preferably a blend of (a) a triglyceride composition comprising OPO and having at least 70% by weight of the palmitic acid content of the composition present as palmitoyl groups bonded at the 2- position and (b) one or more liquid oils, more preferably a mixture of canola (rapeseed) oil, sunflower oil and palm kernel oil. Preferably, the blend comprises 30-60% by weight of (a) and 40-70% by weight of (b).

The triglyceride composition comprising OPO may be produced by a process comprising the reaction of palm oil stearin with oleic acid or an ester thereof in the presence of a lipase.

For example, the triglyceride composition comprising OPO may be produced by a process which comprises:
(i) providing a palm oil stearin comprising tripalmitoyl glyceride and having an iodine value of between about 2 and about 14;
(ii) optionally bleaching and deodorising the palm oil stearin;
(iii) subjecting the palm oil stearin to enzymic transesterification with oleic acid or a non-glyceride ester thereof;
(iv) separating palmitic acid or palmitic non-glyceride esters from the product obtained in (iii); and
(v) optionally dry fractionating the product obtained in (iv) to form a fraction comprising an increased amount of OPO.

The palm oil stearin used in step (i) of the process may be obtained from solvent (wet) fractionation, Lanza fractionation, or dry fractionation of palm oil, such as multi-stage counter current dry fractionation of palm oil. The palm oil can be crude palm oil, refined palm oil, fractions of palm oil (e.g., obtained by dry fractionation), other derivatives of palm oil, or mixtures thereof. The palm oil stearin preferably has a palmitic acid content of at least 60% by weight, more preferably at least 70% by weight, such as at least 75%, at least 80% or at least 85% by weight e.g., at least 90% by weight, based on the total fatty acid residue content. The palm oil stearin used in step (i), preferably comprises 2-palmitoyl glycerides, typically in an amount of greater than 50% by weight, such as greater than 55% by weight or greater than 60% by weight. The palm oil stearin preferably has an iodine value (IV) of between about 2 and about 14, preferably between about 4 and about 14, more preferably between about 6 and about 12, such as from about 8 to about 12. The palm oil stearin is optionally randomly interesterified before or after (ii).

Before the palm oil stearin is subjected to enzymatic esterification, the palm oil stearin is typically refined in optional step (ii), which preferably involves bleaching and deodorising. The bleaching of the palm oil stearin in the process is performed above 95°C, more preferably above 100°C (such as at from 105°C to 120°C). In the deodorising step, volatile impurities are removed from the palm oil stearin to yield deodorised palm oil stearin, typically at temperatures above 200°C. The impurities removed in the deodorising step commonly include free fatty acids, aldehydes, ketones, alcohols and other hydrocarbon impurities. The bleaching and deodorising are performed under standard conditions known in the art and may be carried out in a single process step or two or more process steps. For example, the steps may be carried out at reduced pressures (e.g., 10 mm Hg or below), wherein the palm oil stearin is contacted with steam to help vaporise the impurities. Bleaching and deodorising the palm oil stearin may help to improve the yield of the process.

The enzymic transesterification according to the process is preferably carried out using a 1,3 specific lipase as a biocatalyst.

Under the influence of a 1,3 lipase, unsaturated fatty acid residues may be introduced into the 1- and 3- positions of the 2-palmitoyl glycerides by exchange with the fatty acid residues of other glycerides or more preferably by means of transesterification in the fatty mixture. Exchange preferably takes place between unsaturated free fatty acids, preferably oleic acid, or alkyl esters of oleic acid with alcohols having from 1 to 6 carbon atoms. The 2-palmitoyl glycerides modified in this way may be separated from the reaction mixture.

The enzymic transesterification reaction selectively exchanges palmitic acid with oleic acid on the 1,3-position rather than the 2-position. The transesterification reaction is typically performed to reach or approach equilibrium at a conversion ratio of a minimum of at least 50%, preferably at least 60%, most preferably at least 70%.

Preferably, in the transesterification reaction, the palm oil stearin is, for example, mixed with an oleic acid concentrate (comprising free oleic acid at a concentration of greater than 65% by weight, preferably greater than 70% by weight, most preferably greater than 75% by weight). Alternatively, the oleic acid may be provided as a mixture comprising oleic acid (preferably in an amount of greater than 65% by weight), linoleic acid and, optionally, one or more other fatty acids. The ratio of palm oil stearin to oleic acid concentrate is preferably from 0.1:1 to 2:1, more preferably from 0.4:1 to 1.2:1, even more preferably from 0.4:1 to 1:1, most preferably from 1:1.1 to 1:2 on a weight basis. The reaction is preferably carried out at a temperature of from 30 °C to 90 °C, preferably from 50 °C to 80 °C, such as about 60 °C to 70 °C, and may be conducted batchwise or in continuous fashion, with or without a water-immiscible organic solvent.

Before the enzyme transesterification reaction, the humidity is preferably controlled to a water activity between 0.05 and 0.55, preferably between 0.1 and 0.5, depending on the type of biocatalyst enzyme system used. The reaction may be performed, for example, at 60°C in a stirred tank or in a packed bed reactor over biocatalysts, based on concentrates of Lipase D (*Rhizopus oryzae,* previously classified as *Rhizopus delemar,* from Amano Enzyme Inc., Japan) or immobilised concentrates of *Rhizomucor miehei* (Lipozyme RM IM from Novozymes A/S, Denmark).

In order to separate palmitic acid and other fatty acids or palmitic non-glyceride esters and other glycerides from OPO in (iv), the transesterified mixture (optionally after further treatment, such as isolation of the fat phase) is preferably distilled. Distillation is preferably carried out at low pressure (e.g., lower than 10 mbar) and elevated temperatures (e.g., greater than 200°C) to remove the fatty acids from the product triglyceride fraction.

The composition obtained in (iv) is preferably fractionated in (v) to recover an olein fraction (i.e., a lower melting fraction). This can be done using solvent fractionation, Lanza fractionation or dry fractionation, using a single, two-step or multi-step fractionation technique, but is preferably carried out using single step dry fractionation. The olein can also be obtained by subjecting the transesterified mixture to multi-stage counter current dry fractionation.

Fractionation of the triglyceride fraction removes the unconverted tripalmitin (PPP) down to a level of less than 15 weight %, preferably less than 10 weight %, most preferably less than 8 weight %. The olein fraction, obtained after step (v), is typically further refined or purified to remove remaining fatty acids and contaminants to produce a refined olein fraction.

The process may optionally comprise further steps before, between or after (i) to (v), such as partial purification or enrichment of the products in the desired component(s).

The process may comprise an additional step of further purifying the product in OPO.

The composition obtained after (iv) or (v) preferably comprises at least 10% by weight OPO, more preferably at least 15%, even more preferably at least 20%, such as at least 25% or 30% or even 40% by weight OPO based on total glycerides in the fraction. The balance typically comprises other non-OPO triglycerides, and may further contain minor amounts of diglycerides and monoglycerides. Minor amounts of free fatty acids may also be present. The fraction is preferably a composition which comprises a mixture of triglycerides wherein different fatty acid residues, including unsaturated fatty acid residues, are randomly distributed between the 1- and 3-positions and at least half of the fatty acid residues in the 2-positions are C16 and/or C18 saturated, preferably consisting substantially of palmitic acid residues, in particular 60-90% by weight of the total 2-position fatty acids are preferably palmitic acid. Preferably, all of the fatty acid residues, or virtually all (e.g., greater than 99% by weight), in the glycerides of the composition are even-numbered. The unsaturated fatty acid residues in the 1- and 3-positions preferably consist largely of oleic acid and linoleic acid. The composition preferably includes at least as much (on a molar basis) of saturated fatty acid in the 2-position as in the 1- and 3-positions combined, more preferably up to twice as much (on a molar basis). Preferably, the 1,3-positions include both unsaturated C18 and saturated C4 to C14 fatty acids. The proportion and type of these fatty acids may be determined in accordance with dietary and physical requirements of the composition required.

The composition obtained after (iv) or (v) is preferably blended with at least one vegetable oil to provide the composition for use in the invention. Typically, the amount of vegetable oil is from 40-70% by weight, based on the total weight of the fat blend. Examples of suitable vegetable oils include sunflower oil, high oleic sunflower oil, palm kernel oil, rapeseed oil, flaxseed oil and mixtures thereof.

The composition of the invention is for use in increasing the ratio of fat in subcutaneous adipose tissue to fat in visceral adipose tissue.

In the invention, the glyceride having a palmitoyl group at the sn-2 position increases the ratio of fat in subcutaneous adipose tissue to fat in visceral adipose tissue. Therefore, the invention also provides a method of increasing the ratio of fat in subcutaneous adipose tissue to fat in visceral adipose tissue by oral administration of the glyceride having a palmitoyl group at the 2- position and/or of the composition comprising the glyceride wherein at least 40% by weight of the palmitic acid content of the composition is present as palmitoyl groups bonded at the 2- position of a glyceride.

The invention may involve therapeutic or non-therapeutic use. Therapeutic use may involve the prevention and/or treatment of one or more conditions associated with excess visceral fat relative to fat in subcutaneous adipose tissue.

Excess visceral fat is linked to type 2 diabetes, insulin resistance, inflammatory diseases, and other obesity-related diseases. Therefore, the invention may involve the treatment and/or amelioration of type 2 diabetes, insulin resistance, glucose intolerance, inflammatory diseases, dyslipidaemia, energy and lipid metabolism impairment, ectopic fat accumulation, metabolic disorders (including metabolic syndrome) and other obesity-related diseases by increasing the ratio of fat in subcutaneous adipose tissue to fat in visceral adipose tissue.

Increasing the ratio of fat in subcutaneous adipose tissue to fat in visceral adipose tissue according to the invention may comprise one or more of: optimizing fat distribution to healthy locations; improving the balance between subcutaneous and visceral fat in the body (e.g., of an infant); and helping to balance fat storage.

The composition of the invention is for oral administration, suitably as part of an otherwise normal diet.

A preferred product form for the composition of the invention is an infant formula.

An infant formula composition of the invention may comprise one or more components selected from other fats (i.e., lipids), protein, carbohydrate, minerals and vitamins. The present invention also therefore contemplates an infant formula comprising fat, protein and carbohydrate, for example in the approximate relative weight proportions of fat:protein:carbohydrate of 4-6:1-3:9-15. Dry formulations containing this mixture, together with additional components customary in such formulations may be dispersed in water to produce an emulsion of approximately 2 to 5 grams of fat per 100 ml of dispersion.

Alternatively, the composition may be in the form of a food product, food supplement or pharmaceutical product. The composition optionally comprises a complementary fat. For example, compositions in the form of food products may comprise a complementary fat selected from: cocoa butter, cocoa butter equivalents, palm oil or fractions thereof, palmkernel oil or fractions thereof, interesterified mixtures of said fats or fractions thereof, or liquid oils, selected from: sunflower oil, high oleic sunflower oil, soybean oil, rapeseed oil, cottonseed oil, fish oil, safflower oil, high oleic safflower oil, maize oil and MCT-oils. Food products (which term includes animal feed) contain a fat phase, wherein the fat phase contains the composition of the invention. The food products are suitably selected from the group consisting of: spreads, margarines, creams, dressings, mayonnaises, ice-creams, bakery products, infant food, chocolate, confectionery, sauces, coatings, cheese and soups. Food supplements or pharmaceutical products may be in the form of capsules or other forms, suitable for enteral or parenteral application and comprise a product of the invention.

The listing or discussion of an apparently prior-published document in this specification should not necessarily be taken as an acknowledgement that the document is part of the state of the art or is common general knowledge.

The following non-limiting examples illustrate the invention and do not limit its scope in any way. In the examples and throughout this specification, all percentages, parts and ratios are by weight unless indicated otherwise.

### EXAMPLES

### Example 1

60 male Wistar rats weight 100-150 g were housed for a week before allocating to 3 diets, control (regular chow for rodents), 10 % high Sn-2 diet (LD) and 10 % low Sn-2 tallow based diet (TD) (see Table 1 for fatty acid composition). Rats were fed for 5 weeks. Weight of the animals was recorded weekly, and food intake was recorded every 2 days. The length of the animals was also recorded weekly. Before sacrifice, rats were fasted for 12 h, and killed by decapitation. Liver, adipose tissue and, muscle tissues were taken and frozen at -80°C. The endocannabinoids AEA and 2-AG and the AEA congeners molecules OEA and PEA were measured from total lipid extracts.

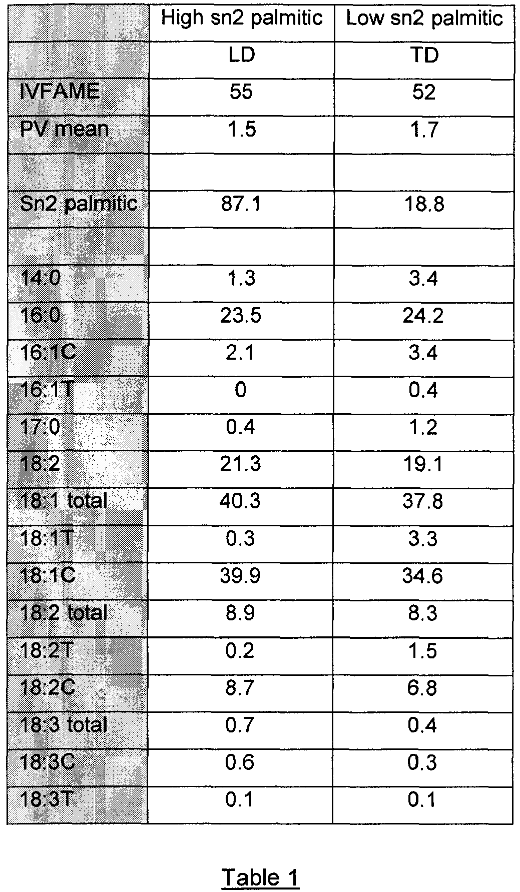

In the above table, IVFAME refers to calculated iodine value, PV is the peroxide value and Cx:y refers to a fatty acid having x carbon atoms and y double bonds. C refers to *cis* fatty acids and T to *trans* fatty acids.

Analyses of lipids included fatty acid profile of TAG and PL fraction and were carried out as described in Banni et al, , Journal of Lipid Research, 2001, 42 (7):. 1056-1061). Endocannabinoids and lipid signalling molecules were measured from total lipid extracts as described in Di Marzo et al, Nature, 2001, 410:(6830):822-825.

### Results

Adipose tissue was analysed for the two major endocannabinoids, anandamide (AEA) and 2-arachidonyl-monoacylglycerol (2-AG) and AEA congeners palmitoylethanolamide (PEA) and oleoylethanolamide (OEA). The data showed statistically significant 50 % lower levels of AEA and 30 % higher level of PEA in the high sn-2 group with respect to the low sn-2 group in visceral adipose tissue as shown in the following Table 2:

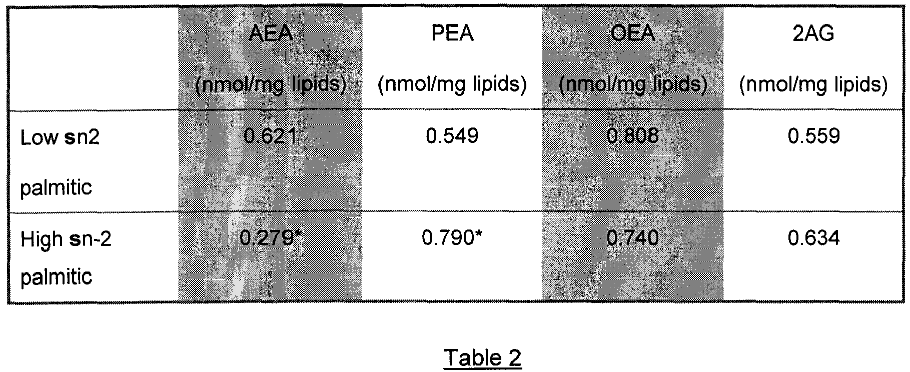

The sum of AEA, PEA and OEA did not differ significantly between the two groups revealing that the biosynthesis and catabolism of acylethanolamides was not affected.

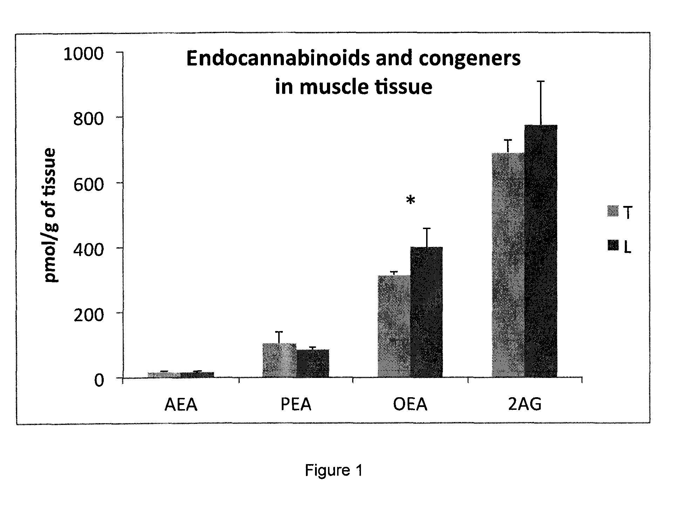

In muscle tissue, oleoylethanolamide (OEA) showed a statistically significant increase of about 25% with the high sn-2 palmitic diet (L) compared to the low sn-2 palmitic diet (T) (Figure 1).

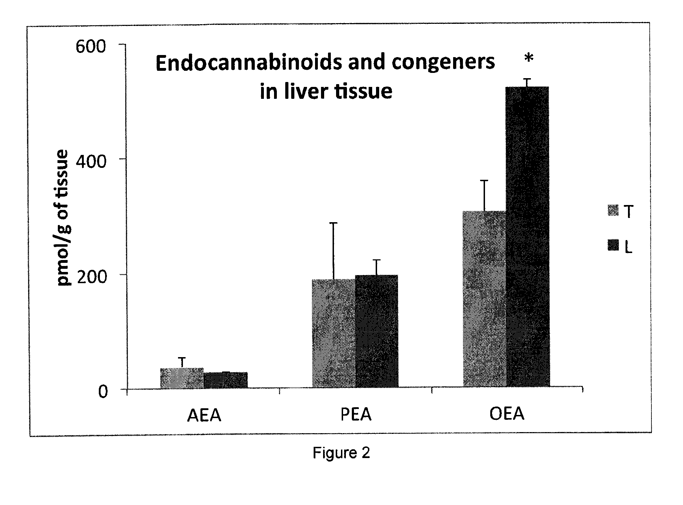

In the liver a similar pattern was found as in the muscle, with higher levels of OEA (Figure 2).

### Example 2

An infant formula is prepared comprising as a fat phase Betapol^{®}45 (Loders Croklaan BV, Wormerveer, The Netherlands) being a fat composition having 47% by weight of the palmitic acid content of the composition present as palmitoyl groups bonded at the 2-position.

## Claims

1. Composition comprising a glyceride having a palmitoyl group at the 2- position wherein at least 40% by weight of the palmitic acid content of the composition is present as palmitoyl groups bonded at the 2- position of a glyceride, for use in increasing the ratio of fat in subcutaneous adipose tissue to fat in visceral adipose tissue.

2. Composition as claimed in Claim 1, for use in increasing the ratio of fat in subcutaneous adipose tissue to fat in visceral adipose tissue by reducing the level of anandamide (AEA) in visceral adipose tissue.

3. Composition as claimed in Claim 1 or Claim 2, for use in increasing the ratio of fat in subcutaneous adipose tissue to fat in visceral adipose tissue by increasing the level of palmitoylethanolamide (PEA) in visceral adipose tissue.

4. Composition as claimed in any one of Claims 1 to 3, wherein the increase in the ratio of fat in subcutaneous adipose tissue to fat in visceral adipose tissue is accompanied by a reduction in fat in visceral adipose tissue.

5. Composition as claimed in any one of Claims 1 to 4, wherein the glyceride having a palmitoyl group is a triglyceride, preferably 1,3-dioleoyl-2-palmitoyl glyceride (OPO).

6. Composition as claimed in any one of Claims 1 to 5, wherein the glyceride having a palmitoyl group is derived from a vegetable source.

7. Composition as claimed in any one of Claims 1 to 6, wherein the glyceride having a palmitoyl group is produced by the reaction of palm oil stearin with oleic acid or an ester thereof in the presence of a lipase.

8. Composition as claimed in any one of Claims 1 to 7, which comprises alpha-linolenic acid or a derivative thereof in an amount of greater than 1% by weight alpha-linoleic acid based on total free and bound C₆ to C₂₄ fatty acids and/or linoleic acid or a glyceride thereof and, wherein the weight ratio of total free and bound linoleic acid to total free and bound alpha-linolenic acid is preferably 15:1 or less.

9. Composition as claimed in any one of Claims 1 to 8 which is a blend comprising (i) a composition comprising alpha-linolenic acid or a glyceride thereof and (ii) a triglyceride composition comprising OPO in an amount of at least 20% by weight.

10. Composition as claimed in Claim 9, wherein the composition comprising alpha-linolenic acid or a glyceride thereof is a vegetable oil.

11. Composition as claimed in any one of Claims 1 to 10, for use in increasing the ratio of fat in subcutaneous adipose tissue to fat in visceral adipose tissue in an infant.

12. Composition as claimed in any one of Claims 1 to 11 which is an infant formula further comprising protein, carbohydrate, minerals and vitamins.

13. Use of a composition comprising a glyceride having a palmitoyl group at the 2-position wherein at least 40% by weight of the palmitic acid content of the composition is present as palmitoyl groups bonded at the 2- position of a glyceride, for increasing the ratio of fat in subcutaneous adipose tissue to fat in visceral adipose tissue.

14. A method of increasing the ratio of fat in subcutaneous adipose tissue to fat in visceral adipose tissue in a subject comprising providing to said subject for consumption a composition comprising a glyceride having a palmitoyl group at the 2- position wherein at least 40% by weight of the palmitic acid content of the composition is present as palmitoyl groups bonded at the 2- position of a glyceride.

15. Use or method of Claim 13 or Claim 14 having the features of any of Claims 2 to 12.
